# EUROPEAN PATENT APPLICATION

(11) **EP 4 484 116 A1**
(43) Date of publication of application: **01.01.2025**
(21) Application number: 24181648.7
(22) Date of filing: 12.06.2024
(51) Int. Cl.: B29C 45/76, G01M 5/00, G01M 99/00, G01N 3/56, G01N 33/00, G01N 33/44, B29L 31/40

(54) **RESIN MOLDED ARTICLE AND METHOD FOR DETERMINING DETERIORATION OF RESIN MOLDED ARTICLE**

(30) Priority: 30.06.2023 JP 2023108199
(71) Applicant: Ricoh Company, Ltd., Tokyo 143-8555 (JP)
(72) Inventor: KAWAMATA, Yuji, Tokyo, 143-8555 (JP)
(74) Representative: J A Kemp LLP

(57) **Abstract**

To easily determine deterioration of a resin molded article without breaking the resin molded article.

A resin molded article 10 made of a predetermined resin material includes a test piece 20 capable of determining deterioration of the resin molded article 10, and a holder portion 11 in which the test piece 20 is detachably installed. Then, the degree of deterioration of the resin molded article 10 is determined using the test piece 20 removed from the holder portion 11.

## Description

### BACKGROUND OF THE INVENTION

### 1. Field of the Invention

The present disclosure relates to a resin molded article made of a predetermined resin material, such as a part constituting an apparatus, and a method for determining deterioration of the resin molded article.

### 2. Description of the Related Art

In various devices (industrial products), resin molded articles (resin parts) made of a predetermined resin material have been used as parts (for example, see Patent Document 1). After such resin molded articles have been used in a terminated first use application and collected thereafter, the degree of deterioration is determined in a recycling factory or the like, and whether the resin molded articles can be reused (reused) is determined based on the degree of deterioration.

Meanwhile, Patent Document 1 discloses a technique for measuring a breaking force when a hole is formed in a semiconductor part storage tray by using a measuring instrument and determining whether the tray can be reused from the measurement result.

The related art resin molded article has been partially broken to determine deterioration and determine whether the molded article can be reused, based on the broken part. Thus, it may be difficult to use the resin molded article as a part such as an exterior cover for which external appearance is regarded as important.

According to the present disclosure, it is desirable to provide a resin molded article and a method for determining deterioration of the resin molded article, which can easily determine deterioration without breakage.

### RELATED ART DOCUMENT

### PATENT DOCUMENT

Patent Document 1: Japanese Patent No. 3998383

### SUMMARY OF THE INVENTION

According to at least one aspect of the present disclosure, a resin molded article made of a predetermined resin material is provided. The resin molded article includes
a test piece capable of determining deterioration of the resin molded article; and
a holder portion in which the test piece is detachably installed,
wherein a degree of deterioration of the resin molded article is determined using the test piece removed from the holder portion.

### ADVANTAGEOUS EFFECT OF THE INVENTION

According to the present disclosure, a resin molded article and a method for determining deterioration of the resin molded article, which can easily determine deterioration without breakage is provided.

### BRIEF DESCRIPTION OF THE DRAWINGS

FIG. 1 is a perspective view illustrating an apparatus according to an embodiment of the present disclosure.
FIG. 2A is a perspective view illustrating a resin molded article in a state where a test piece is mounted.
FIG. 2B is a perspective view illustrating the resin molded article in a state where the test piece is removed.
FIG. 3 is a side sectional view illustrating a state in which the test piece is deformed.
FIGS. 4A and 4B are graphs illustrating a relationship between strain and stress of the test piece.
FIGS. 5A and 5B are perspective views illustrating a resin molded article as a first modification.
FIGS. 6A and 6B are perspective views illustrating a resin molded article as a second modification.
FIG. 7A is a perspective view illustrating a resin molded article as a third modification.
FIG. 7B is a side view illustrating the resin molded article as the third modification as viewed in a direction A.
FIG. 8 is a perspective view illustrating a resin molded article as a fourth modification.
FIGS. 9A and 9B are perspective views illustrating a resin molded article as a fifth modification.
FIGS. 10A and 10B are perspective views illustrating a resin molded article as a sixth modification.
FIG. 11 is a perspective view illustrating an apparatus as a seventh modification.

### DESCRIPTION OF THE PREFERRED EMBODIMENTS

Hereinafter, embodiments for carrying out the present disclosure will be described in detail with reference to the accompanying drawings. In the drawings, the same or corresponding parts are denoted by the same reference numerals, and the overlapping description thereof will be appropriately simplified or omitted.

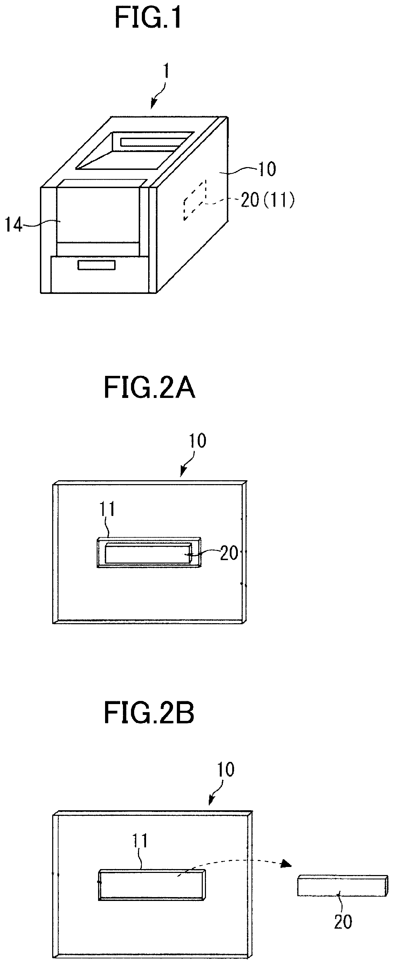

As illustrated in FIG. 1, a reusable resin molded article 10 is installed in an apparatus 1. The resin molded article 10 is made of a predetermined resin material (for example, PC resin, PET resin, ABS resin, or the like). When the first use application of the apparatus 1 is terminated, the apparatus 1 is collected thereafter, and the degree of deterioration of the resin molded article 10, which is disassembled from the apparatus 1 in a recycling factory or the like, is determined. Then, whether the resin molded article 10 can be reused is determined based on the degree of deterioration.

In the present embodiment, an image forming apparatus such as a printer is used as the apparatus 1, but the apparatus 1 is not limited to this image forming apparatus such as a printer, and any industrial product may be used. In the present embodiment, an exterior cover is used as the reusable resin molded article 10, but the reusable resin molded article 10 is not limited to the exterior cover, and any reusable resin molded article may be used. Further, in the present embodiment, the resin molded article 10 is used as a part of the apparatus 1 distributed in the market, but the resin molded article 10 may be used as a product distributed in the market alone. Examples of the industrial products include electronic products such as household electric appliances, and electronic products such as communication devices, transportation devices such as automobiles, vehicles, and elevators, unit articles such as kitchens and bathrooms, buildings such as houses and buildings, civil engineering parts such as road signs, exterior housing parts such as daily sundries, various containers, toys, audio devices, and beauty devices. More specifically, examples of the electric appliances include parts of electric pots, humidifiers, rice cookers, rice washers, coffee makers, dish washers, and dryers, parts of air conditioners, connector housings for electrical connection of wire harnesses of automobiles, and storage trays for semiconductor parts.

Here, referring to FIGS. 2A, 2B, and the like, the resin molded article 10 in the present embodiment is provided with a test piece 20 capable of determining deterioration of the resin molded article 10 and a holder portion 11 in which the test piece 20 is detachably installed. The test piece 20 is a thin, substantially rectangular member made of a resin material having strength deterioration characteristics substantially equal to those of the resin molded article 10. Specifically, in the present embodiment, the test piece 20 is made of the same resin material as the resin molded article 10, and is formed to have substantially the same thickness and substantially the same molding conditions as the resin molded article 10.

Meanwhile, the holder portion 11 is a portion protruding in a recess shape (the inside is depressed in a rectangular parallelepiped shape) so as to be able to lightly hold the test piece 20. The holder portion 11 is made of the same resin material as the resin molded article 10 and is formed as a part of the resin molded article 10 in the same molding process. In addition, the holder portion 11 is formed so as to rise from a non-exterior surface of the resin molded article 10 (which is the inner wall surface that is not exposed when the holder portion 11 is mounted on the apparatus 1 as illustrated in FIG. 1).

Then, referring to FIG. 2B, the degree of deterioration of the resin molded article 10 is determined using the test piece 20 removed from the holder portion 11. Specifically, as illustrated in FIG. 2A, the resin molded article 10 is mounted on the apparatus 1 in a state where the test piece 20 is mounted on the holder portion 11. The resin molded article 10 is then collected from the market to a recycling factory or the like in a state in which the test piece 20 is mounted (the state of FIG. 2A), and the test piece 20 is removed from the resin molded article 10 as illustrated in FIG. 2B. Then, the deterioration determination is performed using the removed test piece 20, and the possibility of reuse of the resin molded article 10 is determined.

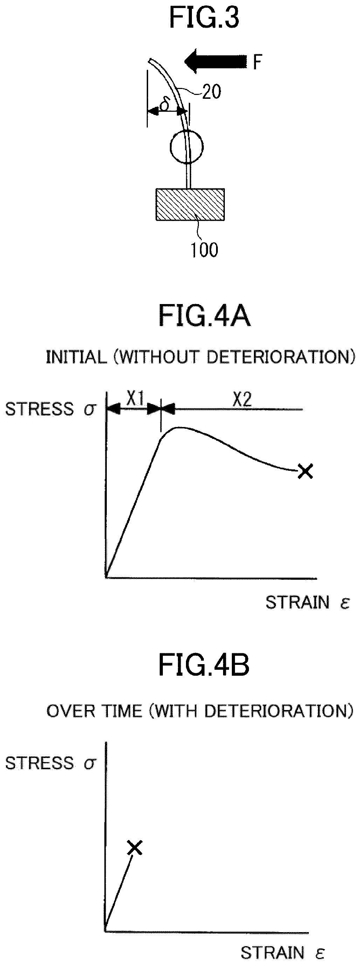

The test piece 20 removed from the resin molded article 10 is supported in a cantilever manner by the jig 100 as illustrated in FIG. 3. In this state, a force F is applied to the test piece 20 by a finger of a person or the like to deform the free end side of the test piece 20. The presence or absence of deterioration (strength deterioration) is determined based on the presence or absence of plastic deformation or fracture. Specifically, the presence or absence of deterioration of the resin molded article 10 is determined by the presence or absence of breakage or plastic deformation when the external force F of a predetermined magnitude is applied to the test piece 20 removed from the holder portion 11 or when the test piece 20 is deformed by a predetermined amount δ. In other words, as the method for determining the deterioration of the resin molded article 10, first, a deformation step of deforming the test piece 20 removed from the holder portion 11 is performed. Then, a determination step of determining that the resin molded article 10 is deteriorated when the test piece 20 is broken or plastically deformed in the deformation step is performed. Specifically, the resin molded article 10 determined to be deteriorated will not be reused, and only the resin molded article 10 determined not to be deteriorated will be reused. When the resin molded article 10 is reused, the test piece 20 (which is not broken or plastically deformed) used for the deterioration determination is set again in the holder portion 11 of the resin molded article 10.

As described above, the resin molded article 10 according to the present embodiment is not determined based on whether the resin molded article can be reused by determining deterioration by breaking or plastically deforming a part of the resin molded article 10, but is determined based on whether the resin molded article can be reused by determining deterioration due to breakage or plastic deformation using the test piece 20 for determining deterioration removed from the resin molded article 10. Therefore, even if the resin molded article 10 is a part for which external appearance is regarded as important, it is possible to easily determine deterioration without causing breakage or plastic deformation and reuse the resin molded article 10 as it is.

In the present embodiment, the test piece 20 is deformed to determine the deterioration of the resin molded article 10, but the test piece 20 may be broken or plastically deformed to determine the deterioration of the resin molded article 10. Specifically, when an external force is applied to the test piece 20 removed from the holder portion 11 until the test piece 20 is broken or plastically deformed, the presence or absence of deterioration of the resin molded article 10 can be determined based on the magnitude of the external force. In such a case, first, a breaking step of applying an external force F to the test piece 20 removed from the holder portion 11 to break the test piece 20 is performed. Then, a determination step is performed to determine whether the resin molded article 10 has deteriorated, based on the magnitude of the external force F when the test piece 20 is broken in the breaking step. Specifically, when the magnitude of the external force F at the time of the occurrence of the breakage in the test piece 20 does not reach a threshold value Fx, the resin molded article 10 is not reused, and when the magnitude of the external force F at the time of the occurrence of the breakage in the test piece 20 exceeds the threshold value Fx, the resin molded article 10 is reused.

FIGS. 4A and 4B are graphs illustrating a relationship between the strain ε and the stress σ in the test piece 20, FIG. 4A illustrating a relationship when the test piece 20 (resin molded article 10) is not deteriorated, and FIG. 4B illustrating a relationship when the test piece 20 (resin molded article 10) is deteriorated. As illustrated in FIG. 4A, the test piece 20 (resin molded article 10) does not have strength deterioration in an initial state, and when force is applied to the test piece 20 (resin molded article 10), the test piece 20 is elastically deformed to a certain stress level (see a range X1), is plastically deformed when the stress level is exceeded, and is finally broken when the deformation is further increased (see a range X2). The stress at the time of plastic deformation or breakage at this time can be generally regarded as the strength of the material (the test piece 20 or the resin molded article 10). In contrast, as illustrated in FIG. 4B, when the test piece 20 (resin molded article 10) is deteriorated in strength due to, for example, a decrease in molecular weight of the resin material by the effect of heat, ultraviolet rays, chemicals, or the like over time, the longitudinal elastic modulus (the slope of the graph in the elastic region) is almost unchanged, but the test piece 20 is likely to be broken suddenly at a stress smaller than that in the non-deteriorated state, with almost no plastic deformation. The strength of the material (the test piece 20 and the resin molded article 10) at this time is smaller than that in a state where there is no deterioration (the state of FIG. 4A).

Here, the resin molded article 10 in the present embodiment is an exterior cover, and as described above, is provided with a non-exterior surface that is not exposed in a state of being mounted on the apparatus 1. In the resin molded article 10, the holder portion 11 and the test piece 20 are disposed on the non-exterior surface. With this configuration, the external appearance of the apparatus 1 is not impaired by the resin molded article 10 provided with the holder portion 11 and the test piece 20.

In the present embodiment, the degree of deterioration of another resin molded article 14 (see FIG. 1) installed in the apparatus in which the resin molded article 10 is installed can also be determined using the test piece 20 removed from the holder portion 11. In the present embodiment, the other resin molded article 14 is an exterior cover installed on the front surface of the apparatus 1, and is made of the same resin material and under the same molding conditions as the resin molded article 10 (exterior cover) installed on the side surface of the apparatus 1. With this configuration, the efficiency of determining whether the parts of the apparatus 1 can be reused as a whole is improved.

### <First Modification>

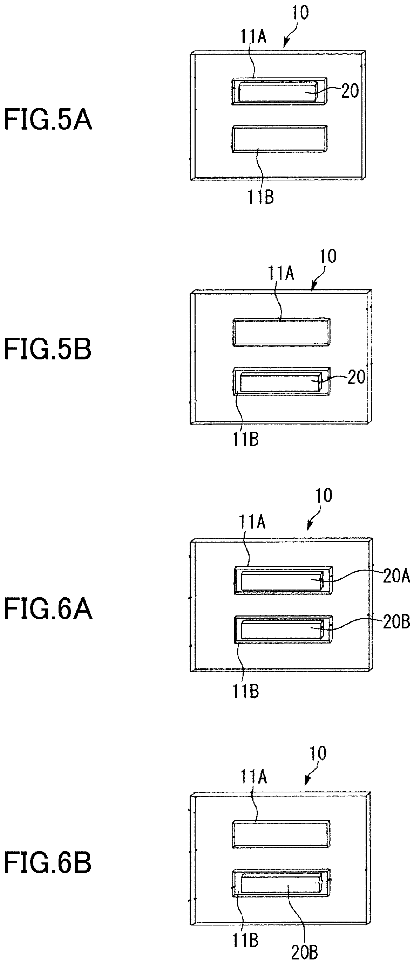

As illustrated in FIGS. 5A and 5B, the resin molded article 10 in the first modification is provided with one test piece 20 and a plurality of holder portions (two holder portions of a first holder portion 11A and a second holder portion 11B in the examples of FIGS. 5A and 5B). As illustrated in FIG. 5A, the test piece 20 is held in the first holder portion 11A of the resin molded article 10 in a brand-new state. Then, the resin molded article 10 that has terminated first use application of the apparatus 1 and has been collected thereafter in a recycling factory or the like is subjected to the deterioration determination of the test piece 20 (resin molded article 10) by using the test piece 20 removed from the first holder portion 11A. When the test piece 20 (resin molded article 10) is determined to be reusable without deterioration, the test piece 20 is set in the second holder portion 11B of the resin molded article 10 to be reused, as illustrated in FIG. 5B. In this way, the use history of the resin molded article 10 can be grasped by changing the position of the holder portion for holding the test piece 20 depending on whether the resin molded article 10 is a new article or a reused article. When a larger number of holder portions are provided, the number of reused times can also be grasped.

### <Second Modification>

As illustrated in FIGS. 6A and 6B, the resin molded article 10 in a second modification is provided with a plurality of test pieces (two test pieces of a first test piece 20A and a second test piece 20B in the examples of FIGS. 6A and 6B) and a plurality of holder portions (two holder portions of a first holder portion 11A and a second holder portion 11B in the examples of FIGS. 6A and 6B), where the number of holder portions is equal to the number of the test pieces (two test pieces of the first test piece 20A and the second test piece 20B).
As illustrated in FIG. 6A, the resin molded article 10 in a brand-new state has the first test piece 20A held by the first holder portion 11A and the second test piece 20B held by the second holder portion 11B. Then, the first use application of the apparatus 1 having the resin molded article 10 is terminated, and the resin molded article 10 that has been collected thereafter in a recycling factory or the like is subjected to the deterioration determination of the first test piece 20A (resin molded article 10) using the first test piece 20A removed from the first holder portion 11A. When the first test piece 20A (resin molded article 10) is determined to be reusable without deterioration, the second test piece 20B is held only by the second holder portion 11B without returning the first test piece 20A to the first holder portion 11A of the resin molded article 10 to be reused, as illustrated in FIG. 6B. In the case of determining whether the reused resin molded article 10 can be reused, the second test piece 20B removed from the second holder portion 11B is used. In this way, the number of test pieces (test pieces 20A and 20B) is changed depending on whether the resin molded article 10 is a new article or a reused article, and thus the use history of the resin molded article 10 can be grasped.

### <Third Modification>

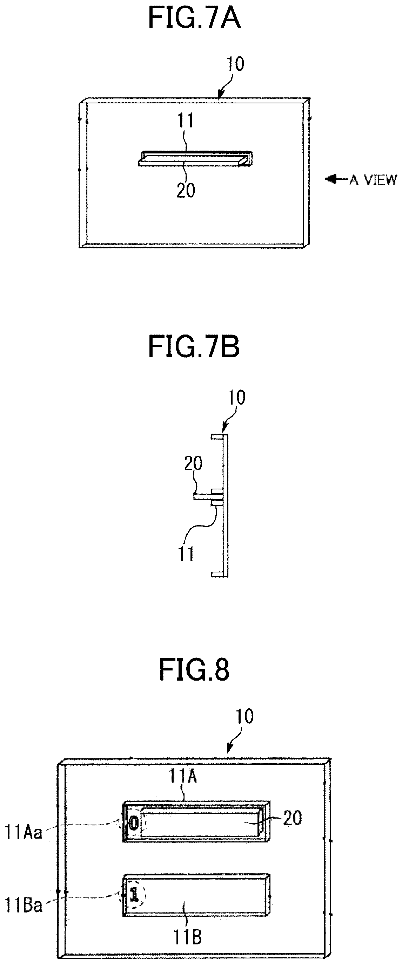

As illustrated in FIGS. 7A and 7B, in the resin molded article 10 according to a third modification, the test piece 20 mounted on the holder portion 11 functions as a rib that reduces deformation of the resin molded article 10. Specifically, the test piece 20 is held by the holder portion 11 such that the longitudinal direction of the test piece 20 is the same as the longitudinal direction of the resin molded article 10 (the horizontal direction in FIGS. 7A and 7B) (such that the longitudinal directions of the test piece 20 and the resin molded article 10 are parallel to each other). The test piece 20 (and the holder portion 11) is disposed so as to rise in a substantially vertical direction from the non-exterior surface of the resin molded article 10. With this configuration, the test piece 20 functions as a reinforcing rib, and thus a defect that the resin molded article 10 warps (deforms) in the longitudinal direction is reduced.

### <Fourth Modification>

As illustrated in FIG. 8, in the resin molded article 10 according to a fourth modification, an indicator for specifying the number of reused times of the resin molded article 10 is provided on at least the holder portions 11A and 11B or the test piece 20 (in the example of FIG. 8, only on the holder portions 11A and 11B). Specifically, the first holder portion 11A is provided with an indicator 11Aa of "0", and the second holder portion B is provided with an indicator 11Ba of "1". These indicators 11Aa and 11Ba are formed by stamping, decal, screen-printing, laser-printing, or the like, and are formed at positions where the indicators 11Aa and 11Ba can be visually recognized even when the test piece 20 is held in the holder portion 11A or 11B. The test piece 20 is held in the first holder portion 11A of the resin molded article 10 in a new product state. Then, the first use application of the apparatus 1 having the resin molded article 10 is terminated and the resin molded article 10 that has been collected thereafter in a recycling factory or the like is subjected to the deterioration determination of the test piece 20 (resin molded article 10) by using the test piece 20 removed from the first holder portion 11A. At this time, an operator recognizes that the deterioration determination of the test piece 20 (resin molded article 10) is the first deterioration determination from the indicator 11Aa of "0" of the first holder portion 11A. When the test piece 20 (resin molded article 10) is determined to be reusable without deterioration, the test piece 20 is set in the second holder portion 11B of the resin molded article 10 to be reused. When determining whether the reused resin molded article 10 can be reused, the second test piece 20B removed from the second holder portion 11B is used, and the operator grasps that the deterioration determination of the test piece 20 (resin molded article 10) has previously been performed once and is the second time from the indicator 11Ba of "1" of the second holder portion 11B. In this way, the use history of the resin molded article 10 can be grasped by changing the position of the holder portion for holding the test piece 20 depending on whether the resin molded article 10 is a new article or a reused article. When the number of the holder portions is increased, the number of reused times can be grasped twice or more.

### <Fifth Modification>

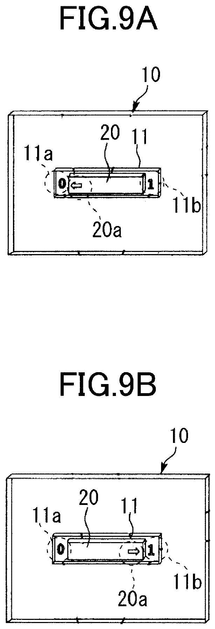

As illustrated in FIGS. 9A and 9B, in the resin molded article 10 according to a fifth modification, an indicator for specifying the number of reused times of the resin molded article 10 is provided on at least one of the holder portion 11 or the test piece 20 (in the examples of FIGS. 9A and 9B, on both the holder portion 11 and the test piece 20). Specifically, the holder portion 11 has an indicator 11a of "0" on one end side in the longitudinal direction and an indicator 11b of "1" on the other end side in the longitudinal direction. The test piece 20 has an indicator 20a (arrow indicator) of "→" at the end in the longitudinal direction. These indicators 11a, 11b, and 20a are formed by stamping, decal, screen-printing, laser printing, or the like. As illustrated in FIG. 9A, the test piece 20 is held by the holder portion 11 of the resin molded article 10 in a brand-new state such that the arrow indicator 20a of the test piece 20 faces the indicator 11a "0" of the holder portion 11. Then, the first use application of the apparatus 1 having the resin molded article 10 is terminated and the resin molded article 10 that has been collected thereafter in a recycling factory or the like is subjected to the deterioration determination of the test piece 20 (resin molded article 10) using the test piece 20 removed from the holder portion 11. At this time, the operator recognizes that the deterioration determination of the test piece 20 (resin molded article 10) is the first time deterioration determination because the indicator 11a of "0" of the holder portion 11 is pointed to by the arrow indicator 20a. When the test piece 20 (resin molded article 10) is determined to be reusable without deterioration, the test piece 20 determined to be reusable is set in the holder portion 11 of the resin molded article 10 to be reused such that the indicator 11b "1" of the holder portion 11 is pointed to by the arrow indicator 20a as illustrated in FIG. 9B. When the reusability of the reused resin molded article 10 is determined, the test piece 20 removed from the holder portion 11 is used. However, since the indicator 11b "1" of the holder portion 11 is pointed to by the arrow indicator 20a, the operator grasps that the deterioration determination of the test piece 20 (resin molded article 10) has previously been performed once and is the second time. In this way, the indicators 11a and 11b indicated by the arrow indicator 20a of the test piece 20 are changed depending on whether the resin molded article 10 is a new article or a reused article, and thus it is possible to grasp the use history of the resin molded article 10.

### <Sixth Modification>

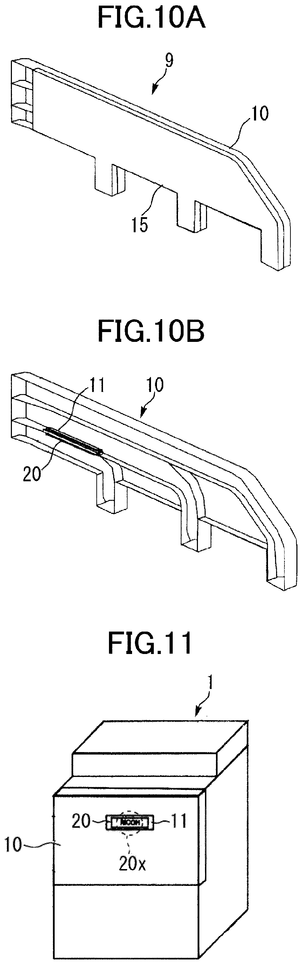

As illustrated in FIGS. 10A and 10B, the resin molded article 10 in a sixth modification is a duct main body in which a partition wall and a flow regulating plate are formed, and is covered with a duct cover 15 as a counterpart component to constitute a duct 9. The duct 9 is installed inside the apparatus 1, and is used to cool the inside of the apparatus 1 by allowing air sucked from the outside of the apparatus 1 to flow into the inside of the apparatus 1. Here, in the sixth modification, the holder portion 11 and the test piece 20 are provided in the resin molded article 10. The holder portion 11 and the test piece 20 are provided in a space (internal space) formed by the duct cover 15 (a counterpart component to be assembled with the resin molded article 10 in the apparatus 1 in which the resin molded article 10 is installed) and the resin molded article 10. Specifically, in the examples of FIGS. 10A and 10B, the test piece 20 and the holder portion 11 are formed as a part of a partition wall forming a duct flow path, and the test piece 20 is detachably installed on the partition wall. In the resin molded article 10 configured as described above, it is possible to easily determine the deterioration of the resin molded article 10 without breaking the resin molded article 10.

### <Seventh Modification>

As illustrated in FIG. 11, in the apparatus 1 according to a seventh modification, the holder portion 11 and the test piece 20 are provided on an outer surface of the resin molded article 10 (the surface of the resin molded article 10 exposed in the state of being mounted on the apparatus 1). Information about the apparatus 1 is displayed on at least one of the holder portion 11 or the test piece 20 (only the test piece 20 in the example of FIG. 11). More specifically, a logo mark 20x "RICOH" is formed on the front side of the test piece 20 by stamping, decal, screen-printing, laser-printing, or the like. Although the logo mark 20x is formed on the surface of the test piece 20 in the sixth modification, a model number or the like may be formed.

As described above, the resin molded article 10 in the present embodiment is a resin molded article made of a predetermined resin material, and is provided with the test piece 20 capable of determining deterioration of the resin molded article 10 and the holder portion 11 in which the test piece 20 is detachably installed. Then, the degree of deterioration of the resin molded article 10 is determined using the test piece 20 removed from the holder portion 11. Thus, the deterioration of the resin molded article 10 can be easily determined without breaking the resin molded article 10.

Note that the present disclosure is not limited to the embodiment, and it is apparent that the embodiment can be altered as appropriate within the scope of the technical idea of the present disclosure, in addition to the modifications suggested in the embodiments. The number, positions, shapes, and the like of the constituent members are not limited to those in the present embodiment, and any number, positions, shapes, and the like suitable for carrying out the present disclosure can be adopted.

Note that aspects of the present disclosure may be a combination of the following Aspects 1 to 14, for example.

### (Aspect 1)

A resin molded article made of a predetermined resin material, comprising:
a test piece capable of determining deterioration of the resin molded article; and
a holder portion in which the test piece is detachably installed,
wherein a degree of deterioration of the resin molded article is determined using the test piece removed from the holder portion.

### (Aspect 2)

The resin molded article according to Aspect 1, wherein the test piece is made of a resin material having strength deterioration characteristics substantially equal to those of the resin molded article.

### (Aspect 3)

The resin molded article according to Aspect 1 or 2,
wherein the resin molded article includes a non-exterior surface that is not exposed in a state of being mounted on an apparatus, and
wherein the holder portion and the test piece are installed on the non-exterior surface.

### (Aspect 4)

The resin molded article according to Aspect 1 or 2,
wherein the resin molded article includes an exterior surface that is exposed in a state of being mounted on an apparatus, and
wherein the holder portion and the test piece are installed on the exterior surface, and information relating to the apparatus is displayed on at least one of the holder portion or the test piece.

### (Aspect 5)

The resin molded article according to any one of Aspects 1 to 4, comprising:
one test piece and a plurality of the holder portions.

### (Aspect 6)

The resin molded article according to any one of Aspects 1 to 4, comprising:
a plurality of the test pieces and a plurality of the holder portions, wherein a number of the plurality of the test pieces is equal to a number of the plurality of the holder portions.

### (Aspect 7)

The resin molded article according to any one of Aspects 1 to 6, wherein the holder portion and the test piece are provided in a space formed by the resin molded article and a counterpart component, the counterpart component being assembled with the resin molded article in an apparatus in which the resin molded article is installed.

### (Aspect 8)

The resin molded article according to any one of Aspects 1 to 7, wherein the test piece in a state of being mounted on the holder portion functions as a rib that reduces deformation of the resin molded article.

### (Aspect 9)

The resin molded article according to any one of Aspects 1 to 8, wherein a degree of deterioration of another resin molded article is also determined using the test piece removed from the holder portion, the another resin molded article being installed in an apparatus in which the resin molded article is installed.

### (Aspect 10)

The resin molded article according to any one of Aspects 1 to 9, wherein at least one of the holder portion or the test piece is provided with an indicator for specifying a number of reused times of the resin molded article.

### (Aspect 11)

The resin molded article according to any one of Aspects 1 to 10, wherein presence or absence of deterioration of the resin molded article is determined by presence or absence of breakage or plastic deformation upon an external force of a predetermined magnitude being applied to the test piece removed from the holder portion or upon the test piece removed from the holder portion being deformed by a predetermined amount.

### (Aspect 12)

The resin molded article according to any one of Aspects 1 to 10, wherein presence or absence of deterioration of the resin molded article is determined by magnitude of an external force upon the external force being applied to the test piece removed from the holder portion until the test piece is broken or plastically deformed.

### (Aspect 13)

A method for determining deterioration of a resin molded article, the resin molded article being made of a predetermined resin material, and the resin molded article including a test piece capable of determining deterioration of the resin molded article and a holder portion in which the test piece is detachably installed, the method comprising:
deforming the test piece removed from the holder portion; and
determining that the resin molded article is deteriorated upon the test piece being broken or being plastically deformed in the deforming.

### (Aspect 14)

A method for determining deterioration of a resin molded article, the resin molded article being made of a predetermined resin material, and the resin molded article including a test piece capable of determining deterioration of the resin molded article and a holder portion in which the test piece is detachably installed, the method comprising:
breaking the test piece removed from the holder portion by applying an external force to the test piece; and
determining whether the resin molded article is deteriorated, based on a magnitude of the external force upon the test piece being broken in the breaking.

### [Description of Symbols]

- 1: Apparatus (Image forming apparatus)
- 9: Duct
- 10: Resin molded article (exterior cover)
- 11: Holder portion
- 11A: First holder portion
- 11B: Second holder portion
- 11Aa, 11Ba, 11a, 11b: Indicator
- 14: Another resin molded article
- 15: Duct cover (mating member)
- 20: Test piece
- 20A: First test piece
- 20B: Second test piece
- 20a: Indicator
- 20x: Logo marks

## Claims

1. A resin molded article made of a predetermined resin material, the resin molded article comprising:
a test piece capable of determining deterioration of the resin molded article; and
a holder portion in which the test piece is detachably installed,
wherein a degree of deterioration of the resin molded article is determined using the test piece removed from the holder portion.

2. The resin molded article according to claim 1, wherein the test piece is made of a resin material having strength deterioration characteristics substantially equal to those of the resin molded article.

3. The resin molded article according to claim 1 or 2,
wherein the resin molded article includes a non-exterior surface that is not exposed in a state of being mounted on an apparatus, and
wherein the holder portion and the test piece are installed on the non-exterior surface.

4. The resin molded article according to claim 1 or 2,
wherein the resin molded article includes an exterior surface that is exposed in a state of being mounted on an apparatus, and
wherein the holder portion and the test piece are installed on the exterior surface, and information relating to the apparatus is displayed on at least one of the holder portion or the test piece.

5. The resin molded article according to claim 1 or 2, comprising:
one test piece and a plurality of holder portions.

6. The resin molded article according to claim 1 or 2, comprising:
a plurality of the test pieces and a plurality of the holder portions, wherein a number of the plurality of the test pieces is equal to a number of the plurality of the holder portions, each of the plurality of the test pieces being the test piece and each of the plurality of the holder portions being the holder portion.

7. The resin molded article according to claim 1 or 2, wherein the holder portion and the test piece are provided in a space formed by the resin molded article and a counterpart component, the counterpart component being assembled with the resin molded article in an apparatus in which the resin molded article is installed.

8. The resin molded article according to claim 1 or 2, wherein the test piece in a state of being mounted on the holder portion functions as a rib that reduces deformation of the resin molded article.

9. The resin molded article according to claim 1 or 2, wherein a degree of deterioration of another resin molded article is also determined using the test piece removed from the holder portion, the another resin molded article being installed in an apparatus in which the resin molded article is installed.

10. The resin molded article according to claim 1 or 2, wherein at least one of the holder portion or the test piece is provided with an indicator for specifying a number of reused times of the resin molded article.

11. The resin molded article according to claim 1 or 2, wherein presence or absence of deterioration of the resin molded article is determined by presence or absence of breakage or plastic deformation upon an external force of a predetermined magnitude being applied to the test piece removed from the holder portion or upon the test piece removed from the holder portion being deformed by a predetermined amount.

12. The resin molded article according to claim 1 or 2, wherein presence or absence of deterioration of the resin molded article is determined by magnitude of an external force upon the external force being applied to the test piece removed from the holder portion until the test piece is broken or plastically deformed.

13. A method for determining deterioration of a resin molded article, the resin molded article being made of a predetermined resin material, and the resin molded article including a test piece capable of determining deterioration of the resin molded article and a holder portion in which the test piece is detachably installed, the method comprising:
deforming the test piece removed from the holder; and
determining that the resin molded article is deteriorated upon the test piece being broken or being plastically deformed in the deforming.

14. A method for determining deterioration of a resin molded article, the resin molded article being made of a predetermined resin material, and the resin molded article including a test piece capable of determining deterioration of the resin molded article and a holder portion in which the test piece is detachably installed, the method comprising:
breaking the test piece removed from the holder portion by applying an external force to the test piece; and
determining whether the resin molded article is deteriorated, based on a magnitude of the external force upon the test piece being broken in the breaking.
